Europäisches Patentamt

(19) European Patent Office

Office européen des brevets

(11) Numéro de publication: **0 063 971
B1**

(12) **FASCICULE DE BREVET EUROPEEN**

(45) Date de publication du fascicule du brevet:
02.10.85

(51) Int. Cl.⁴: **G 01 N 1/10** // G21C17/00

(21) Numéro de dépôt: 82400553.2

(22) Date de dépôt: 26.03.82

(54) Dispositif de prélèvement d'échantillons liquides.

(30) Priorité: 08.04.81 FR 8107064

(43) Date de publication de la demande:
03.11.82 Bulletin 82/44

(45) Mention de la délivrance du brevet:
02.10.85 Bulletin 85/40

(84) Etats contractants désignés:
BE DE GB IT

(56) Documents cités:
DE - A - 2 642 065
DE - A - 2 824 153
FR - A - 1 247 657
FR - A - 2 347 671
GB - A - 878 504
US - A - 4 213 342

(73) Titulaire: **COMMISSARIAT A L'ENERGIE ATOMIQUE
Etablissement de Caractère Scientifique Technique et
Industriel, 31/33, rue de la Fédération, F-75015 Paris (FR)**

(72) Inventeur: **Levos, Christian, Le Hameau Nee -
Hardinvast, F-50690 Martinvast (FR)**
Inventeur: **Perie, Daniel, 1, rue des Ardennes,
F-50100 Cherbourg (FR)**
Inventeur: **Gex, Jean-François, Chemin de la Plaine
Mesline, F-50120 Haineville (FR)**

(74) Mandataire: **Mongrédien, André et al, c/o
BREVATOME 25, rue de Ponthieu, F-75008 Paris (FR)**

BUNDESDRUCKEREI BERLIN

## Description

La présente invention a pour objet un dispositif de prélèvement d'échantillons liquides, et plus particulièrement de liquides radioactifs pour analyse.

La plupart des procédés actuels utilisent un flacon de prélèvement fermé par une membrane souple dans lequel on a préalablement fait le vide. Ce flacon est renversé et piqué à l'extrémité supérieure d'une aiguille creuse dont l'extrémité inférieure est plongée dans le réservoir contenant le liquide à échantillonner: le liquide monte dans le flacon par aspiration. L'extrémité inférieure de l'aiguille creuse peut éventuellement être plongée dans un réservoir intermédiaire qui peut être rempli et vidé par un système de mise sous vide. De telles installations comprennent généralement un nombre élevé de points de prélèvements (jusqu'à 19) disposés circulairement et desservis par un organe à commande hydraulique.

Ces installations présentent un certain nombre d'inconvénients. Tout d'abord leur prix de revient est élevé du fait de la mécanisation poussée pour un nombre limité de prélèvements. D'autre part, après chaque prélèvement il reste toujours des traces du liquide analysé à l'intérieur de l'aiguille creuse et ce liquide résiduel n'évolue pas de la même façon que le liquide contenu dans le réservoir. Dans la plupart de tels dispositifs, il n'est pas possible de rincer les aiguilles et lors d'une prochaine analyse le liquide contenu dans le réservoir risque d'être pollué par les traces restées à l'intérieur de l'aiguille creuse lors de l'analyse précédente: l'échantillon recueilli dans le flacon n'est pas représentatif.

Le document FR-A-2 347 671 décrit un dispositif comportant une boucle permettant de faire circuler le liquide à échantillonner à travers un dispositif semblable à un tube de Venturi. Un flacon est relié par deux aiguilles creuses à deux points de ce dispositif où la pression du liquide est différente, ce qui créé une circulation à l'intérieur du flacon. Si un tel dispositif permet un rinçage du flacon et des aiguilles, il reste relativement complexe par la nécessité d'utiliser une pompe pour faire circuler le liquide et la présence d'une conduite de retour.

La présente invention a justement pour objet un dispositif de prélèvement d'échantillons liquides, notamment radioactifs, qui permet un bon rinçage du flacon et des aiguilles tout en étant encore plus simple que les dispositifs selon l'art intérieur.

Le dispositif objet de l'invention comporte, de manière connue, des moyens pour supporter un flacon de prélèvement fermé de manière étanche par un bouchon ou une membrane souple, des moyens de liaison mettant ledit flacon en communication avec un réservoir contenant le liquide à échantillonner, lesdits moyens de liaison comprenant une première aiguille creuse reliant le flacon de prélèvement au liquide à échantillonner par l'intermédiaire d'un premier conduit, une deuxième aiguille creuse, débouchant aussi dans le flacon de prélèvement, et des moyens permettant de créer une différence de pression pour aspirer le liquide provenant du réservoir et l'amener dans le flacon de prélèvement. Selon l'invention, ce dispositif est caractérisé en ce qu'il comporte en un deuxième flacon dit flacon laveur, la deuxième aiguille creuse reliant le flacon de prélèvement au flacon laveur par l'intermédiaire d'un deuxième conduit, en ce que la première aiguille creuse débouche dans le flacon de prélèvement à un niveau inférieur à celui où débouche la deuxième aiguille creuse, et en ce que lesdits moyens permettant de créer une différence de pression sont agencés de manière à créer une différence de pression entre le réservoir et le flacon laveur.

Selon un premier aspect du dispositif objet de l'invention, les moyens permettant de créer une différence de pression entre le réservoir et le flacon laveur comprennent un conduit reliant ce dernier à un système d'aspiration, ledit conduit étant muni d'un dispositif de fermeture.

Selon un second aspect du dispositif objet de l'invention, la partie inférieure du flacon laveur est située à un niveau supérieur à celui de la partie supérieure du réservoir contenant le liquide à échantillonner.

On remarque immédiatement que, grâce au dispositif de l'invention, il n'est plus nécessaire de créer un vide préalable dans le flacon de prélèvement et que, en outre, le système d'aspiration permet un fonctionnement en »continu«. Ce fonctionnement continu provoque un rinçage préalable des conduits et des aiguilles et donc la prise d'un échantillon représentatif.

De toute façon l'invention sera mieux comprise à la lecture de la description qui va suivre, donnée à titre d'exemple purement illustratif et nullement limitatif, en référence aux dessins annexés, dans lesquels:

— la figure 1 est une vue schématique en coupe de l'ensemble du dispositif objet de l'invention; et

— la figure 2 est une vue agrandie, schématique et en coupe, montrant de manière plus détaillée le flacon de prélèvement et le flacon laveur.

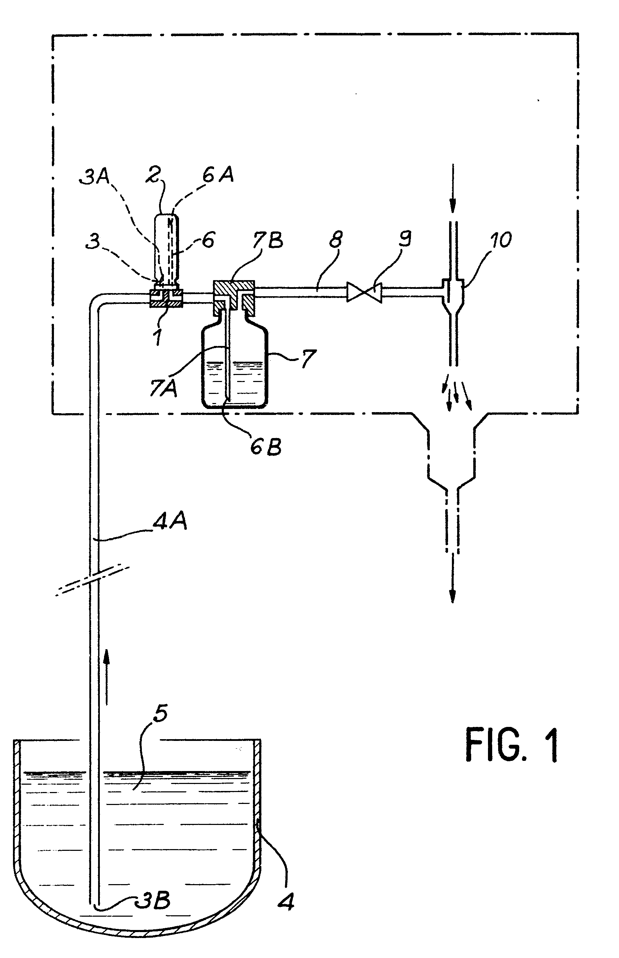

La figure 1 représente l'ensemble du dispositif objet de l'invention. On voit tout d'abord un support 1 sur lequel est placé le flacon de prélèvement 2. Le support 1 est traversé par une première aiguille creuse 3 dont une extrémité 3A passant à travers le support 1 pénètre à l'intérieur du flacon de prélèvement 2 et qui se prolonge par un conduit 4A dont l'extrémité 3B plonge dans un réservoir 4 contenant le liquide à échantillonner 5. Une deuxième aiguille creuse 6 a une extrémité 6A qui débouche dans le flacon 2 après passage à travers le support 1; elle se prolonge par un conduit 7A qui traverse un dispositif 7B fermant de manière étanche la partie supérieure d'un deuxième flacon dit »flacon laveur« 7:

l'extrémité 6B du conduit 7A débouche au fond de ce deuxième flacon. La contenance de ce flacon laveur 7 est supérieure à celle du flacon de prélèvement 2. L'intérieur du flacon 7 est mis en communication à travers le dispositif 7B avec une conduite 8 munie d'un système de fermeture 9, par exemple une vanne, et qui aboutit à un système d'aspiration 10. Ce dernier peut être une trompe à eau ou un éjecteur à air permettant de créer une différence de pression entre le réservoir 4 et le flacon laveur 7.

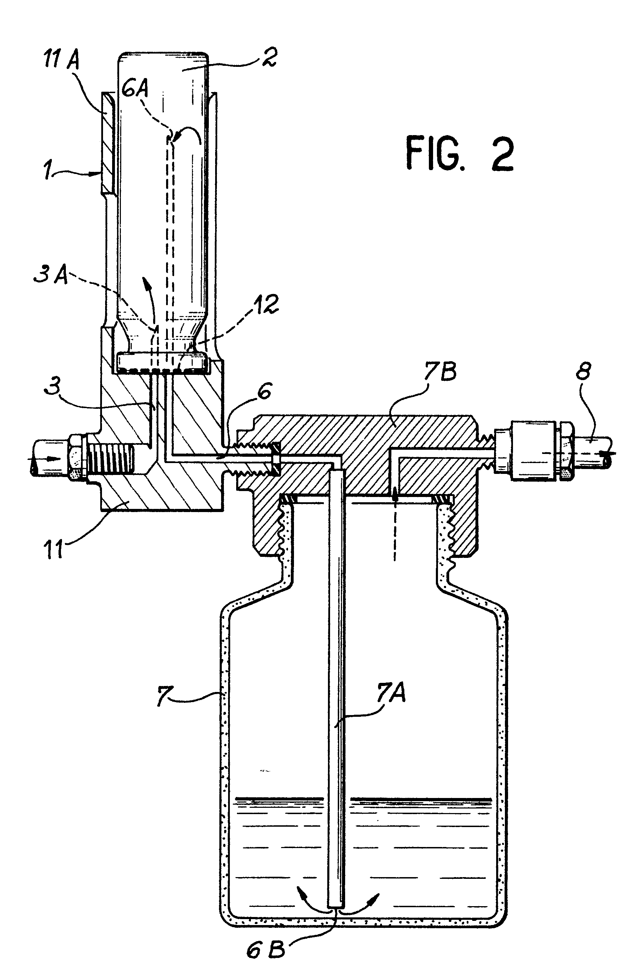

La figure 2 montre plus en détail le dispositif servant de support au flacon de prélèvement 2. Le support 1 comporte une partie inférieure 11 pleine à travers laquelle passent les deux aiguilles creuses 3 et 6. Celles-ci débouchent à l'intérieur d'une partie cylindrique creuse 11A prolongeant la partie inférieure 11. Le flacon de prélèvement, fermé de manière étanche par un bouchon ou une membrane souple 12, est introduit à l'intérieur de la partie cylindrique creuse 11A. Le flacon 2 est renversé de sorte qu'au cours de cette opération, la membrane 12 soit percée par les deux aiguilles 3 et 6. Les parois de la partie cylindrique creuse 11A sont suffisamment ajourées pour que l'on puisse contrôler le niveau du liquide à l'intérieur du flacon 2.

Le fonctionnement de l'appareil est le suivant:

Lorsqu'on vient de placer le flacon de prélèvement 2 sur le support 1, le flacon laveur 7 étant vide, on ouvre la vanne 9 et l'on met en route le système d'aspiration 10. Ceci crée une dépression à l'intérieur du flacon laveur 7 et, par aspiration, le liquide 5 contenu dans le réservoir 4 monte le long du conduit 4A et de l'aiguille 3 jusque dans le flacon de prélèvement 2. Bien entendu, pour que ceci soit possible, il faut que le flacon laveur 7 et le flacon de prélèvement 2 soient situés à un niveau supérieur à celui de la partie supérieure du réservoir 4. Il faut prendre garde également à ce que la différence de hauteur de liquide entre le réservoir 4 et le flacon laveur 7 représente une différence de pression inférieure au vide produit par la trompe à eau. A l'intérieur du flacon 2, l'extrémité 3A de l'aiguille 3 se trouve à un niveau inférieur à celui de l'extrémité 6A de l'aiguille 6: cette disposition est nécessaire pour que le liquide à analyser arrivant par l'aiguille 3 remplisse au moins partiellement le flacon de prélèvement 2 avant de s'écouler dans le flacon laveur 7 à travers l'aiguille 6 et le conduit 7A.

La présence du flacon laveur 7 s'explique par la nécessité de renouveler plusieurs fois le contenu du flacon 2 pour avoir un échantillon représentatif. En effet, même dans le dispositif objet de l'invention, il reste des traces de liquide dans l'aiguille 3 et le conduit 4A après une analyse, d'où la nécessité de les rincer avant l'analyse suivante. Dans un exemple de réalisation, où la différence de hauteur entre le réservoir 5 et le flacon laveur 7 était de 6,50 m et la capacité du flacon de prélèvement de 10 ml, on a constaté qu'il fallait renouveler au moins dix fois le contenu du flacon de prélèvement pour avoir un échantillon représentatif. Le flacon laveur 7 sert à stocker le trop-plein provenant de cette opération.

Une fois que le flacon 2 est rempli d'un échantillon représentatif, on ferme la vanne 9 et on arrête la trompe à eau 10.

Le flacon 2 est alors retiré et remplacé par un flacon vide. Pendant l'intervalle de temps séparant l'enlèvement du premier flacon et la mise en place du deuxième, les quantités de liquide contenues dans l'aiguille 3 et le conduit 4A d'une part, l'aiguille 6 et le conduit 7A d'autre part, redescendent par gravité dans le réservoir 4 et le flacon 7 respectivement.

Pour récupérer le liquide contenu dans le flacon 7 et le recycler dans le réservoir 4, on opère de la manière suivante:

Tout d'abord, on réamorce le circuit en ouvrant la vanne 9 et en mettant en route la trompe à eau 10 jusqu'à ce que le nouveau flacon de prélèvement soit rempli de liquide. On arrête ensuite la trompe à eau 10 mais sans fermer la vanne 9: la surface du liquide contenu dans le flacon laveur 7 est alors soumise à la pression atmosphérique de même que celle du liquide 5 contenu dans le réservoir 4. Comme la surface du liquide contenu dans le flacon 7 est à un niveau supérieur à celui de la surface du liquide 5, et comme le flacon 2 est rempli de liquide entre les extrémités 3A et 6A des aiguilles 3 et 6, il se produit un phénomène de siphonnage et tout le liquide contenu dans le flacon 7 redescend automatiquement dans le réservoir 4. Bien entendu, pour que cela soit possible, il faut que le conduit 7A débouche dans le flacon 7 le plus bas possible vers le fond de celui-ci. Le dispositif est alors prêt pour un nouveau prélèvement.

Le dispositif objet de l'invention présente de nombreux avantages et tout d'abord une grande simplicité puisqu'il ne fait appel à aucun automatisme, ni système de régulation de vide. Les différents éléments constituant le dispositif sont peu coûteux et facilement disponibles dans le commerce. D'autre part, la distance entre le dispositif et le réservoir n'a pas d'importance car il suffit que la différence de niveau entre le flacon laveur et le réservoir de liquide à échantillonner représente une différence de pression inférieure au vide produit par la trompe à eau: c'est ainsi qu'on a pu constater qu'un dispositif conforme à l'invention fonctionnait avec une dénivellation de 7,50 m et une longueur de tuyauterie de 80 m. De plus, le tropplein de liquide recueilli dans le flacon 7 lors du rinçage de l'aiguille 3 peut être facilement renvoyé au réservoir 4 par siphonnage après chaque prélèvement. On supprime ainsi le risque de rejeter dans le circuit de la trompe à eau, ou dans l'atmosphère si on utilise un éjecteur à air, des effluents radioactifs ou chargés de matières fissiles. Quant au deuxième flacon servant au siphonnage, il peut être laissé en place pour l'analyse suivante si la solution ne risque pas de cristalliser au cours du temps.

## Revendications

1. Dispositif de prélèvement d'échantillons liquides, notamment radioactifs, comportant des moyens (1) pour supporter un flacon de prélèvement (2) fermé de manière étanche par un bouchon ou une membrane souple (12), des moyens de liaison mettant ledit flacon (2) en communication avec un réservoir (4) contenant le liquide à échantillonner (5), lesdits moyens de liaison comprenant une première aiguille creuse (3) reliant le flacon de prélèvement (2) au liquide à échantillonner (5) par l'intermédiaire d'un premier conduit (4A), une deuxième aiguille creuse (6) débouchant aussi dans le flacon de prélèvement (2) et des moyens (9, 10) permettant de créer une différence de pression pour aspirer le liquide provenant du réservoir (4) et l'amener dans le flacon de prélèvement (2), ledit dispositif étant caractérisé en ce qu'il comporte un deuxième flacon dit flacon laveur (7), la deuxième aiguille creuse (6) reliant le flacon de prélèvement (2) au flacon laveur (7) par l'intermédiaire d'un deuxième conduit (7A), en ce que la première aiguille creuse (3) débouche dans le flacon de prélèvement (2) à un niveau inférieur à celui où débouche la deuxième aiguille creuse (6), et en ce que lesdits moyens (9, 10) permettant de créer une différence de pression sont agencés de manière à créer une différence de pression entre le réservoir (4) et le flacon laveur (7).

2. Dispositif selon la revendication 1, caractérisé en ce que lesdits moyens permettant de créer une différence de pression comprennent un conduit (8) reliant le flacon laveur (7) à un système d'aspiration (10), ledit conduit étant muni d'un dispositif de fermeture (9).

3. Dispositif selon la revendication 2, caractérisé en ce que la partie inférieure du flacon laveur (7) est située à un niveau supérieur à celui de la partie supérieure du réservoir (4) contenant le liquide à échantillonner (5) et en ce que sa contenance est supérieure à celle du flacon de prélèvement (2).

## Patentansprüche

1. Einrichtung zur Probennahme von Flüssigkeiten, insbesondere radioaktiven, mit Mitteln (1) zum Haltern einer Entnahmeflasche (2), die dichtend mit einem weichen Stöpsel oder einer weichen Membrane (12) geschlossen ist, Verbindungsmitteln, die die Flasche (2) mit einem die Probenflüssigkeit (5) enthaltenden Behälter (4) verbinden, wobei die Verbindungsmittel eine erste Hohlnadel (3), die über eine erste Leitung (4A) die Entnahmeflasche (2) mit der Probenflüssigkeit (5) verbindet, eine zweite Hohlnadel (6), die ebenfalls in die Entnahmeflasche (2) mündet, und Mittel (9, 10) aufweist, die einen Druckunterschied erzeugen können, um die von dem Behälter (4) kommende Flüssigkeit anzusaugen und sie in die Entnahmeflasche (2) einzubringen, dadurch gekennzeichnet, daß die Einrichtung eine zweite, Waschflasche (7) genannte Flasche aufweist, daß die zweite Hohlnadel (6) die Entnahmeflasche (2) mittels einer zweiten Leitung (7A) mit der Waschflasche (7) verbindet, daß die erste Hohlnadel (3) in die Entnahmeflasche (2) auf einer Höhe mündet, die unter derjenigen liegt, wo die zweite Hohlnadel (6) mündet, und daß die Mittel (9, 10), die die Erzeugung eines Druckunterschiedes ermöglichen, derart ausgebildet sind, daß ein Druckunterschied zwischen dem Behälter (4) und der Waschflasche (7) erzeugt wird.

2. Einrichtung nach Anspruch 1, dadurch gekennzeichnet, daß die Mittel, die die Erzeugung eines Druckunterschiedes ermöglichen, eine Leitung (8) umfassen, die die Waschflasche (7) mit einem Saugsystem (10) verbindet, wobei diese Leitung mit einer Verschlußeinrichtung (9) versehen ist.

3. Einrichtung nach Anspruch 2, dadurch gekennzeichnet, daß sich der untere Abschnitt der Waschflasche (7) auf einer Höhe befindet, die höher als der obere Abschnitt des die Probenflüssigkeit (5) enthaltenden Behälters (4) ist, und daß das Fassungsvermögen der Waschflasche (7) größer als dasjenige der Entnahmeflasche (2) ist.

## Claims

1. Apparatus for taking liquid samples, especially radioactive samples, comprising means (1) for supporting a sample flask (2) closed in fluid tight manner by a stopper or a flexible membrane (12), connection means placing said flask (2) in communication with a reservoir (4) containing the liquid (5) to be sampled, said connection means comprising a first hollow needle (3) connecting the sample flask (2) to the liquid (5) for sampling by means of a first conduit (4A), a second hollow needle (6) also opening into the sample flask (2), and means (9, 10) enabling the creation of a pressure difference for aspiration of the liquid from the reservoir (4) and delivering it to the sample flask (2), said apparatus being characterized in that it comprises a second flask (known subsequently as the »wash bottle«) (7), the second hollow needle (6) connecting the sample flask (2) to the wash bottle (7) through a second conduit (7A), and in that the first hollow needle (3) opens into the sample flask (2) at a level below that at which the second hollow needle (6) opens, and in that said means (9, 10) enabling the creation of a pressure difference are operated in a manner to produce a pressure difference between the reservoir (4) and the wash bottle (7).

2. Apparatus according to Claim 1, characterized in that said means enabling the creation of a pressure difference comprise a conduit (8) connecting the wash bottle (7) to an aspiration system (10), said conduit being provided with a closure means (9).

3. Apparatus according to Claim 2, characterized in that the lower part of the wash bottle (7) is located at a level above that of the other part of the reservoir (4) containing the liquid (5) for sampling, and in that its capacity is greater than that of the sample flask (2).

FIG. 1

FIG. 2